# EUROPEAN PATENT APPLICATION

(11) **EP 4 663 135 A1**
(43) Date of publication of application: **17.12.2025**
(21) Application number: 25182698.8
(22) Date of filing: 13.06.2025
(51) Int. Cl.: A61B 17/00

(54) **A CLOSURE DEVICE FOR VASCULAR PUNCTURE**

(30) Priority: 14.06.2024 CN 202410770427
(71) Applicant: Shanghai Kegang Medical Technology Co., Ltd, Shanghai Songjiang District 201601 (CN)
(72) Inventor: RU, Chengtao, Shanghai, 201601 (CN); YU, Ganzhou, Shanghai, 201601 (CN); ZHU, Hongwei, Shanghai, 201601 (CN)
(74) Representative: Cameron Intellectual Property Ltd

(57) **Abstract**

A tissue closure device for vascular puncture sites, comprising a proximal temporary closure device, a pusher device that slides axially along a main catheter, an implant, and a main handle controlling the device. The proximal temporary closure device can change shape and diameter size. The pusher device on the outside of the main catheter slides axially along to approach or move away from the device. The implant is mounted on the main catheter and slides with the pusher device. The implant can expand within tissue at the puncture site to achieve hemostasis. The main handle controls the shape of the device through a traction wire. The main catheter inner diameter houses a rear sleeve, having a multi-sided radiating tubular structure with a central circular cavity with an inner diameter larger than the diameter of the traction wire, allowing the traction wire to slide within the rear sleeve.

## Description

### CROSS-REFERENCE TO RELATED APPLICATIONS

This application claims priority from Chinese Patent Application No. CN202410770427 filed June 14, 2024.

### FIELD OF THE INVENTION

The present application relates to the field of medical devices, particularly to a closure device for vascular puncture.

### BACKGROUND

Interventional procedures have become relatively common and the preferred choice for many doctors and patients in hospitals today due to their minimal invasiveness, operational convenience, and lower patient risk, among other reasons. Common procedures such as coronary and peripheral angiography, interventional ablation for atrial fibrillation, angioplasty, cerebral angiography, and heart valve repair/replacement all utilize interventional techniques. These procedures involve guiding specially designed catheters, guidewires, and other precision instruments into the human body under the guidance of medical imaging equipment to diagnose and locally treat pathological conditions. Consequently, interventional procedures generally require vascular puncture in or of the patient to establish a surgical pathway using the blood vessel as a channel for introducing instruments like catheters and guidewires. In many interventional procedures, patients need anticoagulants or related drugs to reduce risks associated with blood clotting. For example, patients undergoing atrial fibrillation ablation typically take anticoagulants, and patients with coronary thrombosis are administered thrombolytic drugs. While these drugs are used for treatment and complication prevention, they also make hemostasis at the puncture site difficult. Patients undergoing such procedures usually need to remain immobile for over five hours post-operation to prevent complications like subcutaneous hematoma or bleeding caused by puncture site bleeding. However, prolonged immobility often causes significant discomfort and inconvenience to patients and even poses substantial risks of other diseases, such as venous thrombosis, which can lead to more severe conditions like pulmonary embolism. Furthermore, diseases requiring interventional procedures predominantly affect the elderly, for whom these difficulties and complications pose greater risks and higher probabilities. Therefore, hemostasis and sealing of the vascular puncture site after interventional procedures become crucial.

Several methods currently exist to address the above issues. In clinical practice, when patients lack normal coagulation function and the puncture site cannot close naturally through coagulation, common methods include manual suturing by doctors and devices that simulate manual suturing to close the puncture site. However, these methods are complex to operate, requiring significant effort and time from the physician to suture the puncture site effectively. Devices simulating suturing are also inconvenient due to their operational complexity. Additionally, suturing carries the potential risk of causing vascular stenosis.

Among existing products, Cardiva Medical's vascular closure device, VASCADE, is relatively common in United States cardiovascular surgery treatments and is also designed to solve the above problems. However, it has the following problem: The implant of this device is located at the proximal end of the device. When the device enters the human tissue and before temporary occlusion (i.e., sealing the vascular puncture site) is performed, the implant has already come into contact with blood within the tissue tract, causing it to swell. This swelling makes it difficult to deploy the implant from the protective sheath, leading to sealing failure.

In the VASCADE product, the mechanism controlling the deployment of the front-end temporary closure device is a mechanical limit snap-fit mechanism located at the distal end of the device. During use, pulling this mechanism backward deploys the front-end temporary closure device. The mechanism uses a weak limit snap-fit structure to fix the relative distance pulled back. To retract the temporary closure device, the resistance of the snap-fit must be overcome by pushing the mechanism proximally. Since operators typically wear gloves during surgery, which are relatively smooth, using this mechanism requires forcefully pinching it to increase friction before pulling it backward. This process also requires overcoming the snap-fit resistance, making it very inconvenient.

Chinese Patent No. CN202410334089.9 discloses a vascular puncture site closure device. It comprises a proximal temporary closure device, a push device capable of sliding axially, an implant, and a main handle located distally for controlling the temporary closure device. The deployment and retraction of the temporary occlusion are controlled by applying or releasing force via the handle. This force acts on the temporary occlusion through a traction wire connected to it, passing through a main conduit. The main conduit is a single-layer conduit. This device has inherent drawbacks: Because the main conduit is single-layered and the traction wire passes through it, there is a significant gap between the traction wire and the inner diameter of the main conduit, allowing substantial room for deviation. When the temporary occlusion is deployed, the traction wire transmits the force applied by the handle to the temporary occlusion. The tension transmitted by the traction wire can cause it to deviate from the central position of the main conduit. At this point, the structure and working principle of the traction wire and main conduit resemble those of a steerable sheath - the further the traction wire deviates from the center, the more severe the bending of the main conduit. Simultaneously, because the main conduit bends, a significant portion of the force from the handle is exerted on the bent conduit rather than on the temporary occlusion. Consequently, to deploy the temporary occlusion, the force required from the handle is larger than expected. Moreover, the greater the force on the traction wire, the more severe the bending of the main conduit. This principle results in severe bending of the main conduit and excessive handle force. The bending affects the intended functionality of the product, the high handle force makes operation difficult, and it poses a higher risk of failure.

This solution has another problem. The temporary occlusion part of the vascular puncture site closure device relies on the deformation of a braided structure to cause a change in diameter, thereby achieving occlusion. Due to the deformation of the temporary occlusion, the internal volume of the temporary occlusion changes. The volume increases during the transition from the original state to the occluded state and decreases during the transition back from the occluded state to the original state. Since the outer layer of the temporary occlusion is covered by a sealed film, the only pathway for gas exchange within the temporary occlusion is through the inner lumen of the conduit. During deployment, as the internal volume of the temporary occlusion increases, gas can be forcibly drawn in through the narrow inner lumen of the conduit due to the support of the braided structure and the applied force. However, when the temporary occlusion transitions back from the occluded state to the original state, the space shrinks, and the braided structure retracts. The outer covering film, adhered only at the proximal and distal ends of the temporary occlusion, cannot retract simultaneously with the braided structure. Furthermore, because the film is relatively thin and has low stiffness, it cannot provide sufficient retraction force to forcibly expel the gas present during deployment through the narrow inner lumen (which may have a reinforcing tube). This results in a significant volume of gas remaining within the film of the temporary occlusion when it transitions from the deployed state back to the original state. The entire film remains inflated (like an inflated balloon) for an extended period. Since the vascular closure device is used inside blood vessels within the human body, the inflated film can cause difficulties during retraction and lead to serious problems like dislodging the implant, posing significant failure risks and safety hazards.

### SUMMARY

The present disclosure is to solve the problem in existing devices where the film is prone to inflation, causing difficult retraction and dislodging the implant. A tissue closure device is provided for vascular puncture sites that facilitates the expulsion of gas from within the film, enabling smooth retraction.

To achieve this tissue closure device for a vascular puncture site, the device includes:
A proximal temporary closure device capable of actively changing its shape and size in diameter.
A push device, configured as a hollow tubular structure, slidably mounted over a main conduit and capable of sliding axially along the main conduit to approach or move away from the temporary closure device.
An implant, configured as a hollow tubular structure mounted on the main conduit, following the push device to slide axially along the main conduit. This implant is capable of expanding within the tissue at the puncture site to achieve hemostasis.
A distal main handle capable of controlling the shape change of the temporary closure device via a traction wire.
A rear conduit is provided within the inner diameter of the main conduit. The rear conduit is a polygonal radial-shaped tube with a central lumen. The lumen is circular, has an inner diameter larger than the diameter of the traction wire, and allows the traction wire to slide within the rear conduit.

The inner diameter of the rear conduit lumen is greater than the diameter of the traction wire by 0.01mm to 0.5mm. The number of radial arms in the polygonal radial shape of the rear conduit is at least greater than three. The number of radial arms in the rear conduit is preferably an odd number. There should be sufficient gaps between the radial arms of the rear conduit. When the rear conduit is nested within an outer conduit, the radial arms serve as supports for fixation, keeping the traction wire relatively centered. The gaps between the radial arms allow gas to flow smoothly, expelling excess gas when the temporary occlusion is retracted.

Preferably, the rear conduit is a multi-lumen tube. It has a central lumen that is circular with an inner diameter larger than the traction wire diameter. Other lumens, serving as gas pathways, are distributed around the central lumen.

The temporary closure device comprises a segment of braided structure with an attached covering film. The proximal and distal ends of the braided structure have external limiting rings for fixation. These rings clamp the braided structure to prevent it from unraveling. An inner conduit is provided inside the main conduit. The outer diameter of the inner conduit is smaller than the inner diameter of the external limiting rings. The inner conduit can be nested inside the external limiting rings and inside the braided structure. The inner conduit extends into the space inside the external limiting rings and the braided structure. The rear conduit and the inner conduit can be connected end-to-end or can be spaced apart by a distance.

The beneficial effect of the present device is that it provides a rapid and safe tissue closure device for vascular puncture sites. The rear conduit with radial arms fixes the traction wire, and the gaps between the radial arms allow gas to flow smoothly, expelling excess gas when the temporary occlusion is retracted. This facilitates the expulsion of gas from within the film, enabling smooth retraction. It makes hemostasis at the vascular puncture site after interventional procedures fast, efficient, and reduces complications. It provides a rapid and safe method for closing the puncture site and achieving hemostasis for populations in need, such as the elderly or individuals with coagulation dysfunction.

The features and advantages of the present device will be more apparent and easier to understand as shown in the preferred embodiments, described in detail below with reference to the accompanying drawings.

### BRIEF DESCRIPTION OF THE DRAWINGS

To more clearly illustrate the specific embodiments of the present device or the technical solutions in the prior field, the drawings required for describing the specific embodiments or the prior field will be briefly introduced below. Obviously, the drawings in the following description are some embodiments of the present device. For those skilled in the field, other devices may also be obtained from these drawings without creative efforts.
Fig. 1 is a schematic view of an embodiment of the present device.
Fig. 2 is a detailed cross-sectional view of the present temporary occlusion device.
Fig. 3 is a schematic diagram showing the state of the inner conduit structure of the present device when the temporary occlusion is deployed.
Fig. 4 is a cross-sectional schematic view of one embodiment of the rear conduit of the present device.
Fig. 5 is a cross-sectional schematic view of another embodiment of the rear conduit of the present device.

### DETAILED DESCRIPTION

The following provides a further detailed description of the present device to enable those skilled in the field to implement it based on the explanatory text.

The present disclosure relates to a device suitable for individuals with coagulation dysfunction, enabling convenient and safe sealing and hemostasis of vascular puncture sites. It comprises: a proximal temporary closure device, a push device capable of sliding axially, an implant, and a main handle located distally for controlling the temporary closure device. The proximal temporary closure device can actively change its morphological dimensions in diameter (e.g., expand or contract). The push device is a hollow tubular structure capable of sliding axially to approach or move away from the temporary closure device. The implant is a hollow tubular structure that slides axially along with the push device. This implant can expand within the tissue at the puncture site, utilizing the principle of physical expansion for hemostasis. Simultaneously, the implant can be absorbed by the human body without leaving any residue. The distal main handle can control the morphological change of the temporary closure device through simple pressing operations.

The proximal temporary closure device is a structure capable of changing in diameter. It mainly consists of a covering membrane, a braided structure covered by the membrane, and a central traction wire. The covering membrane is a barrel-shaped film, preferably made of a polymer material with high fracture elongation, such as thermoplastic elastomer (TPE), polyvinyl chloride (PVC), polyethylene (PE), silicone, etc. The braided structure, completely covered by the membrane, is preferably made of nitinol wire, stainless steel wire, copper wire, or other metal wires, or polymer plastic wires such as nylon. The braided structure is preferably a one-over-one braid, which ensures good deployment integrity and is relatively simple to manufacture. The wire diameter of the braid is preferably between 0.05mm and 0.3mm, as this range provides suitable deployment force, stable deployed structure, and resistance to external damage. The pitch of the braid is preferably between 5mm and 80mm, its length is preferably between 2mm and 50mm, and the number of braiding spindles (carriers) is preferably between 15 and 60. Process experience shows that the designed deployed diameter corresponds to a relatively specific optimal pitch. To achieve the desired deployed diameter, the pitch must reach this corresponding optimal value, and the length must similarly reach its corresponding optimal length; otherwise, the deployed structure will be incomplete and prone to damage. The diameter of the braided structure is preferably between 0.5mm and 10mm, determined according to the design; in this device, this diameter must match the inner diameter of the vascular access sheath.

The proximal end (starting end) and distal end (terminal end) of the braided structure have outer limiting rings for fixation. These rings clamp the braided structure, preventing it from unraveling. The inner diameter of the outer limiting ring is preferably 1mm smaller to 1mm larger than the diameter of the braided structure. This provides sufficient space for the braid while preventing excessive looseness that could lead to instability. Their thickness is preferably less than 1mm, and length is preferably between 0.5mm and 5mm. This thickness and length facilitate manufacturing, meet functional requirements, and avoid occupying excessive space. Inside the starting end outer limiting ring is an inner limiting ring. The outer and inner limiting rings together clamp and fix the braided structure between them. The length of the inner limiting ring is similar to that of the outer limiting ring. The outer diameter of the inner limiting ring is preferably 5mm to 0.5mm smaller than the outer diameter of the outer limiting ring, and its inner diameter is preferably between 0.1mm and 5mm; the reasoning for these preferred parameters is similar to that for the outer limiting ring. The materials for the inner and outer limiting rings are preferably nitinol alloy, iron alloy, copper, etc. Inside the inner limiting ring is a support tube, used to support the axial deformation of the braided structure during deployment. The outer diameter of the support tube is smaller than the inner limiting ring. It is a hollow structure with an inner diameter preferably between 0.01mm and 2mm. The support tube inner diameter must be larger than the traction wire, ensuring the nitinol wire can pass through, while providing sufficient strength to resist bending under force. The support tube length is slightly longer than the entire braided structure. Its material is preferably nitinol alloy or iron alloy. Inside the support tube is the traction wire, which runs through the entire device. Its length is preferably between 10mm and 500mm, and its diameter is preferably between 0.05mm and 2mm. The traction wire must ensure sufficient strength not to break under the required tensile force while maintaining some flexibility. Its material is preferably nitinol alloy or iron alloy. At the proximal starting end of the temporary closure device, the outer limiting ring, braided structure, inner limiting ring, support tube, and traction wire are all connected and fixed together at the starting end. Preferably, these components are welded or glued together.

The main conduit is a nested structure consisting of three tubes: an outer tube, an inner tube, and a rear tube. The outer tube is the outermost layer with the largest outer and inner diameters among the three tubes. Preferably, its outer diameter is comparable to the inner diameter of the outer limiting ring, with a difference of 0-1mm. This facilitates bonding without significant steps, reducing the risk of injuring the patient's vessel wall during use. The inner diameter of the outer tube is larger than the outer diameter of the inner tube, allowing the inner tube to nest inside the outer tube. The outer diameter of the inner tube is preferably 0-0.5mm smaller than the inner diameter of the outer limiting ring, allowing it to nest inside the outer limiting ring. More preferably, the inner tube can nest inside the outer limiting ring and the braided structure. The inner tube extends into the outer limiting ring and the braided structure, preferably by 1-10mm. This design offers two main benefits: First, the distal end (terminal end) outer limiting ring, braided structure, and main conduit (inner and outer tubes) are bonded together. The inner tube extending inside the outer limiting ring prevents adhesive from incorrectly bonding to the support tube, which needs to slide freely within the inner tube. This structure ensures the support tube's free sliding. Second, the extension length of the inner tube controls the degree of temporary seal deployment. When deployed, the starting end of the temporary seal and the support tube move together towards the terminal end of the temporary seal. Simultaneously, the braided structure, compressed in length, expands radially. The closer the starting and terminal ends move, the more fully the braided structure expands. Third, this stepped structure aids in positioning during product assembly. Since the inner tube extends into the temporary seal, the closest the starting and terminal ends can get is when the starting end's inner limiting ring contacts the inner tube. Therefore, the extension length of the inner tube limits the maximum deployment of the temporary seal; the deeper the inner tube extends, the less the temporary seal can expand. The end face of the outer tube contacts the end face of the outer limiting ring, which aids assembly positioning. Preferably, the end face of the outer tube is processed to have a notch shape. This facilitates adhesive penetration, enhancing bond strength. The length of the inner tube within the outer tube must be greater than the distance the support tube moves inward during temporary seal deployment, at least 10mm greater, preferably between 10mm and 50mm. The rear tube is a polygonal radial tubular shape (sunburst-shaped) or a multi-lumen tube. Preferably, the rear tube is polygonal radial tubular (sunburst-shaped). It should have a central inner lumen, circular in shape, with an inner diameter larger than the traction wire diameter, preferably close to it (0.01mm to 0.5mm larger). The radial outer diameter of the rear tube is smaller than the inner diameter of the outer tube, allowing the rear tube to nest inside the outer tube. The number of radial arms must be at least three to provide stable support within the outer tube without wobbling. An odd number of arms is preferred to minimize wobble space by avoiding symmetry. Preferably, the number of arms is 5, 7, or 9. Sufficient gaps should exist between the arms. When the rear tube is nested within the outer tube, the arms act as supports, keeping the traction wire relatively centered, while the gaps between the arms allow gas to vent smoothly, expelling excess gas when the temporary seal is retracted.

The rear tube can also be a multi-lumen tube. It has a central lumen, circular in shape, with an inner diameter larger than the traction wire diameter (preferably close to it, 0.01mm to 0.5mm larger). Other lumens, serving as gas pathways, surround the central lumen; their cross-sections can be circular or irregular.

Preferably, the rear tube is nested inside the outer tube. The inner diameter of the outer tube must be larger than the outer diameter of the rear tube. The rear tube can butt against the inner tube or be spaced apart from it.

The push device can slide over the main conduit, moving within the range between the temporary closure device and the main handle. The push device generally consists of a protective tube, a secondary handle, and a push rod. The protective tube is a thin-walled tubular structure that protects the implant inside. Its length covers the implant and push rod, and its distal end is bonded to the secondary handle. Its material is preferably a polymer such as polyethylene, nylon, or polyimide. The proximal end of the protective tube has a slightly constricted structure to prevent unintended implant dislodgement. At this constriction, the protective tube is split into two halves to facilitate active implant release. Inside the proximal end of the protective tube is the implant location. Adjacent to the implant is the push rod. The distal end of the push rod abuts the secondary handle. When the secondary handle is operated to push, it applies force to the push rod, which transmits the pushing action to the implant, thereby advancing it. The push rod is also a hollow tube, with an outer diameter smaller than the protective tube and an inner diameter larger than the main conduit. Its material is preferably a polymer. The secondary handle has a through-hole for sliding on the main conduit. Its distal end has a buckle structure for connecting to the main handle. The proximal end of the secondary handle is bonded to the protective tube.

The implant, protective tube, push rod, and secondary handle can all slide on the main conduit. The main conduit is equipped with an anti-reverse ring. Its function is to prevent the push rod from being withdrawn once it has passed the ring, and it also serves to limit the final push position of the push device. The anti-reverse ring is a ring-shaped structure bonded to the main conduit, with an outer diameter smaller than the inner diameter of the push rod. The distal end of the push rod has a constricted structure where its inner diameter gradually decreases to be smaller than the outer diameter of the anti-reverse ring. At this constriction, the push rod is split into two halves. This structure, cooperating with the anti-reverse ring, prevents the push rod from being withdrawn after passing the ring. The process of pushing the collagen implant mainly involves two actions: pushing and withdrawing the protective tube. Correspondingly, the device has three states: pre-push state, push-completed state, and protective tube withdrawn state. The pre-push state is the initial position before pushing. The secondary handle is connected to the main handle, and the implant, push rod, and protective tube are all relatively distal. The anti-reverse ring is located near the proximal end of the push rod. When the push action begins, the operator pushes the secondary handle. The secondary handle separates from the main handle, and the entire push device moves proximally. During this motion, the distal end of the push rod passes over the anti-reverse ring. When the push rod contacts the anti-reverse ring (or the secondary handle contacts a stop), the push limit is reached, ending the push action. This is the push-completed state. The implant is positioned near the temporary closure device, but its proximal end maintains a safe distance from the distal end of the temporary closure device. This safety distance ensures the implant does not enter the blood vessel and is preferably between 0.5mm and 10mm. Next, the protective tube withdrawal action is performed to expose the implant, allowing it to contact tissue fluid and expand. To withdraw the protective tube, the operator retracts the patient's procedural sheath, simultaneously pulling the secondary handle distally. The secondary handle and protective tube retract, exposing the implant. Meanwhile, the push rod is blocked by the anti-reverse ring and cannot retract, keeping the implant and push rod in the push-completed position. When the secondary handle retracts and reconnects to the main handle, the withdrawal is complete, and the vascular sheath is removed from the patient's tissue.

The implant is shaped like a hollow cylinder and can slide on the main conduit. In the pre-push state, it is located inside the protective tube. Its length is preferably between 2mm and 30mm, determined by the thickness of the target human tissue, and should be slightly shorter than that thickness. The implant material is preferably an absorbable, biodegradable material that expands upon contact with liquid, such as collagen or polyethylene glycol. A key characteristic is that after absorbing water and expanding, it can transform from a hollow cylinder into a solid column without a central hole. During expansion, the side walls of the hollow cylindrical implant split open and expand radially, simultaneously pushing the main conduit to the outside of the implant. This facilitates device removal without dislodging the implant. The expansion time upon contact with liquid is preferably between approximately 5s and 100s. The expanded volume is about 2 to 10 times the original volume.

The technical solutions in the embodiments of the present device will be described clearly and completely below in conjunction with the accompanying drawings. Obviously, the described embodiments are only a part of the embodiments of the present device, not all of them. Components of the embodiments, generally described and illustrated in the drawings herein, may be arranged and designed in various different configurations. Therefore, the following detailed description of the embodiments provided in the drawings is not intended to limit the scope of the claimed device but merely represents selected embodiments of the present device. Based on the embodiments presented, all other embodiments obtained by those skilled in the field without creative efforts shall fall within the protection scope of the present claims.

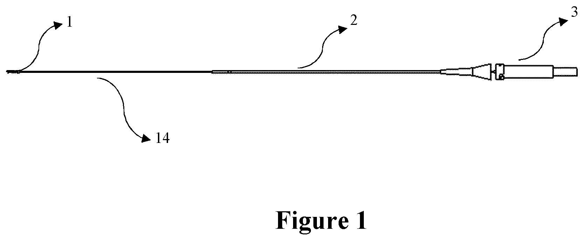

Please refer to FIG. 1, which is a schematic diagram of an embodiment of the present invention. As shown in FIG. 1, the device mainly consists of a temporary closure device (1), a push device (2), and a main handle (3).

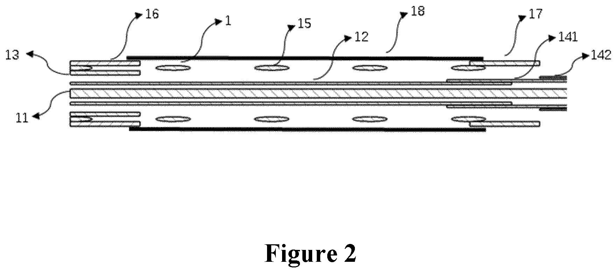

FIG. 2 is a detailed cross-sectional view of the temporary closure device. Within the temporary closure device (1), from the inside out, the innermost layer is the traction wire (11), followed by the support tube (12), the inner limiting ring (13), the main conduit assembly (14), the braided structure (15), the proximal outer limiting ring (16), the distal outer limiting ring (17), and the covering membrane (18). The proximal components - traction wire (11), support tube (12), inner limiting ring (13), braided structure (15), and proximal outer limiting ring (16) - are welded together at the front end. The distal components - inner tube (141), main conduit (142), braided structure (15), and distal outer limiting ring (17) - are bonded together at the distal end.

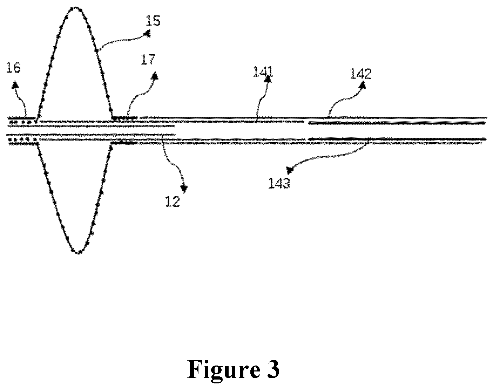

FIG. 3 is a schematic diagram of the inner tube structure during deployment of the temporary seal. As shown in FIG. 3: inner tube (141), main conduit (outer tube) (142), rear tube (143). The rear tube (143), inner tube (141), and main conduit (outer tube) (142) form a stepped structure. The inner tube (141) extends into the distal outer limiting ring (17) and nests inside it, surrounding the support tube (12). The main conduit (outer tube) (142) rests against the outside of the braided structure (15) and distal outer limiting ring (17). The stepped structure formed by the inner tube (141) and main conduit (outer tube) (142) aids positioning during assembly. The extension of the inner tube (141) inside the outer limiting ring (17) prevents adhesive from incorrectly bonding to the support tube (12), which needs to slide freely within the inner tube (141). The length the inner tube extends controls the degree of temporary seal deployment. When deployed, the proximal starting end (16) of the temporary seal and the support tube (12) move together towards the distal end (17) of the temporary seal. Simultaneously, the braided structure (15), compressed in length, expands radially. The closer the starting end (16) and terminal end (17) move, the more fully the braided structure (15) expands.

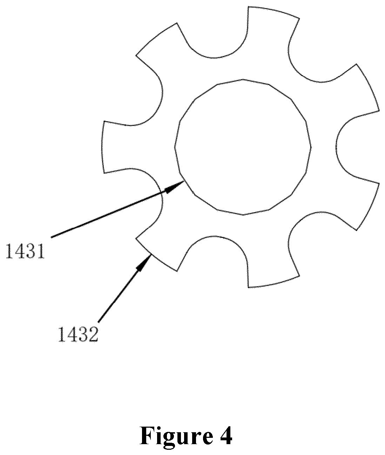

FIG. 4 is a cross-sectional schematic diagram of one embodiment of the rear tube of the present invention. The rear tube is a polygonal radial tubular shape (sunburst-shaped) or a multi-lumen tube. Preferably, it is polygonal radial tubular (sunburst-shaped). The rear tube should have a central inner lumen (1431), circular in shape, with an inner diameter larger than the traction wire diameter (preferably close to it, 0.01mm to 0.5mm larger). The radial outer diameter of the rear tube is smaller than the inner diameter of the outer tube, allowing the rear tube to nest inside the outer tube. The number of radial arms (1432) must be at least three to provide stable support within the outer tube without wobbling. An odd number of arms (1432) is preferred to minimize wobble space by avoiding symmetry. Preferably, the number of arms (1432) is 5, 7, or 9. Sufficient gaps should exist between the arms (1432). When the rear tube is nested within the outer tube, the arms (1432) act as supports, keeping the traction wire relatively centered. The gaps between the arms (1432) allow gas to flow smoothly, expelling excess gas when the temporary seal is retracted.

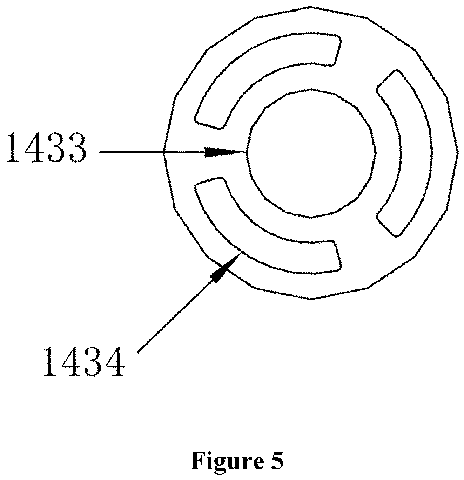

FIG. 5 is a cross-sectional schematic diagram of another embodiment of the rear tube of the present invention. The rear tube can also be a multi-lumen tube. It has a central lumen (1433), circular in shape, with an inner diameter larger than the traction wire diameter (preferably close to it, 0.01mm to 0.5mm larger). Other lumens (1434), serving as gas pathways, surround the central lumen (1433); their cross-sections can be circular or irregular.

Although specific embodiments of the present device have been disclosed above, the device is not limited solely to the descriptions and implementations listed in the specification. It can be fully applied to various fields suitable for the present device. Additional modifications can be readily implemented by those skilled in the field. Therefore, without departing from the general concepts defined by the claims and their equivalents, the present device is not limited to the specific details and illustrations shown and described herein.

## Claims

1. A tissue closure device for a vascular puncture site, comprising: a proximal temporary closure device; a delivery device slidably movable along the axis of a main conduit; an implant; and a main handle located distally for controlling the temporary closure device; wherein the proximal temporary closure device is capable of actively changing its morphological size in diameter; the delivery device is a hollow tubular structure sleeved over the exterior of the main conduit and is slidably movable along the axis of the main conduit towards or away from the temporary closure device; the implant is a hollow tubular structure enveloping the main conduit, moves slidably along the axis of the main conduit following the delivery device, and is capable of expanding within tissue at the puncture site to achieve hemostasis; the distal main handle controls the morphological change of the temporary closure device via a traction wire; **characterized in that** the inner diameter of the main conduit is provided with a rear sleeve, the rear sleeve being a polygonal radially-shaped tube, having a central lumen which is circular in shape, the inner diameter of said lumen being larger than the diameter of the traction wire, and the traction wire being slidable within the rear sleeve.

2. The tissue closure device for a vascular puncture site according to claim 1, **characterized in that** the inner diameter of the lumen of the rear sleeve is 0.01 mm to 0.5 mm larger than the diameter of the traction wire.

3. The tissue closure device for a vascular puncture site according to claim 1, **characterized in that** the number of radiating arms of the rear sleeve is at least greater than three.

4. The tissue closure device for a vascular puncture site according to claim 1, **characterized in that** the number of radiating arms of the rear sleeve is an odd number.

5. The tissue closure device for a vascular puncture site according to claim 1, **characterized in that** the rear sleeve has sufficient space between its radiating arms; when the rear sleeve is nested within an outer sleeve, the radiating arms serve as supports for fixation, maintaining the traction wire in a relatively central position, and the spaces between the radiating arms allow for the smooth or even venting of gases to expel excess gas generated during the retraction of the temporary closure device.

6. The tissue closure device for a vascular puncture site according to claim 1, **characterized in that** the rear sleeve is a multi-lumen tube, wherein it has a central lumen which is circular in shape with an inner diameter larger than the diameter of the traction wire, and other lumens serving as gas pathways, said other lumens being distributed around the central lumen.

7. The tissue closure device for a vascular puncture site according to claim 1, **characterized in that** the temporary closure device is a segment of external braided structure, covered with a layer of covering material; the proximal and distal ends of the segment of external braided structure are provided with external limiting rings serving a fixation function, and the external limiting rings constraining the segment of external braided structure to prevent it from unraveling.

8. The tissue closure device for a vascular puncture site according to claim 7, **characterized in that** the main conduit is internally provided with an inner sleeve; the outer diameter of the inner sleeve is smaller than the inner diameter of the external limiting ring; the inner sleeve can be nested within the interior of the external limiting ring and within the external braided structure; the inner sleeve extends into the space within the external limiting ring and the external braided structure.

9. The tissue closure device for a vascular puncture site according to claim 8, **characterized in that** the rear sleeve is either abutted against the inner sleeve or spaced apart from it by an interval.
